# FASCICULE DE BREVET EUROPEEN

(11) **EP 0 576 380 B1**
(45) Date de publication et mention de la délivrance du brevet: **13.11.1996**
(21) Numéro de dépôt: 93420277.1
(22) Date de dépôt: 25.06.1993
(51) Int. Cl.: A61M 39/00

(54) **Système de connexion stérile**
Steriles Verbindungssystem
Sterile connection system

(30) Priorité: 26.06.1992 FR 9208125
(43) Date de publication de la demande: 29.12.1993
(73) Titulaire: LABORATOIRE AGUETTANT, 69007 Lyon (FR)
(72) Inventeur: Frezza, Pierre, F-69390 Vourles (FR)
(74) Mandataire: Guerre, Dominique

(56) Documents cités:
- WO-A-88/06895
- FR-A- 1 155 487
- US-A- 3 986 508
- US-A- 4 512 766
- US-A- 4 534 758

## Description

La présente invention est relative à un système de connexion stérile entre trois tuyaux, destiné à la circulation dans des conditions stériles d'un milieu liquide ou gazeux, par exemple un liquide corporel tel que du sang, un médicament à administrer à un patient, par exemple une solution perfusée, ou encore un sérum physiologique.

Plus précisément, mais de manière non exclusive, l'invention sera décrite dans le cadre d'un système de dialyse ambulatoire (DPCA), permettant au malade de se dialyser lui-même, au lieu de l'hémodialyse pratiquée obligatoirement en milieu hospitalier.

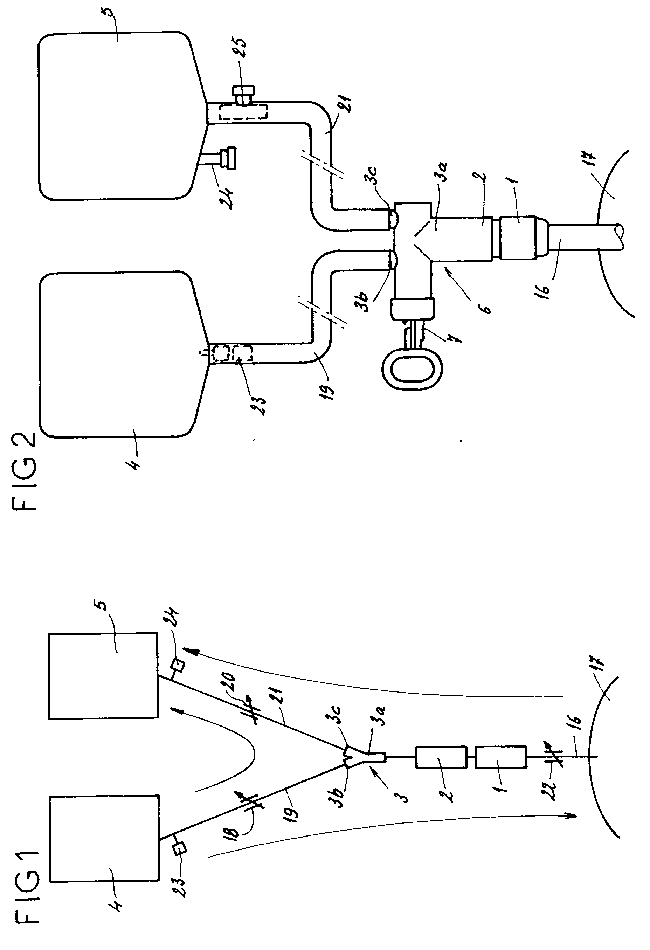

A ce jour, on connaît le système suivant de connexion stérile, décrit par référence à la figure 1, comportant :
- un premier connecteur 1 relié par un tuyau ou drain 16, implanté par voie chirurgicale sur le corps du patient, à la cavité péritonéale 17 de ce dernier
- un deuxième connecteur 2, agencé de manière complémentaire au premier connecteur 1 pour établir d'une manière réversible une liaison ou communication avec ce dernier
- un organe 3 de liaison à trois branches, dont une première 3a est reliée au deuxième connecteur 2, une seconde 3b par un robinet de contrôle 18 sur un tuyau 19, à une réserve 4 ou poche pleine de solution de dialyse, et une troisième 3c, par un robinet de contrôle 20 sur un tuyau 21, à un réservoir 5 ou poche vide destinée à recevoir le dialysat
- un robinet de contrôle 22, disposé sur le drain, entre le premier connecteur 1 et la cavité péritonéale 17
- éventuellement, un site d'injection 23 en relation avec la poche pleine 4 de solution de dialyse, et un site d'extraction 24, en relation avec la poche vide 5 pour le dialysat, ceci de manière à pouvoir ajouter ou prélever un échantillon sur la solution de dialyse et le dialysat respectivement.

Tandis que le premier connecteur 1 est fixé à demeure sur la cavité péritonéale, en liaison avec le drain 16 et le robinet 22, même si ces éléments sont implantés et changés périodiquement, l'ensemble constitué par le deuxième connecteur 2, la jonction 3 à trois branches, la poche pleine 4 et la poche vide 5, est jetable et conditionné dans un emballage stérile.

La mise en oeuvre de ce système de connexion stérile est la suivante, en partant d'un patient dont le dialysat doit être évacué :
- au départ les trois robinets 18, 20 et 22 étant fermés, le deuxième connecteur 2 est branché sur le premier connecteur 1
- la poche vide ou réservoir 5 étant en position basse pour recueillir le dialysat, les robinets 20 et 22 sont ouverts, et le dialysat s'écoule alors par gravité vers la poche vide 5
- la poche 5 à dialysat étant remplie, on ferme le robinet 22 sur le drain du patient et on ouvre le robinet 18 vers la poche pleine 4, disposée en hauteur, moyennant quoi la solution de dialyse s'écoule vers la poche 5 à dialysat, et rince la ligne d'écoulement vers le patient
- puis on ferme le robinet 20 vers la poche à dialysat, et on ouvre le robinet 22 sur le drain du patient, moyennant quoi la solution de dialyse s'écoule vers la cavité péritonéale 17
- lorsque la réserve 4 de solution de dialyse est vide, on ferme le robinet 22 sur le drain du patient, et on débranche le deuxième connecteur 2 du premier 1, et l'ensemble ainsi constitué comme indiqué précédemment est jeté.

Conformément au document WO-A-88 06895, on a proposé de rassembler et intégrer les robinets 18, 20 et 22 décrits précédemment, dans un seul et même distributeur rotatif entre les trois branches 3a, 3b, 3c décrites selon figure 1. Ce distributeur est susceptible de prendre quatre positions, commandées par l'utilisateur, à savoir :
* une position de purge, dans laquelle les seconde et troisième branches sont ouvertes et communiquent entre elles, et la première branche est fermée
* une position de recueil, dans laquelle les première et troisième branches sont ouvertes et communiquent entre elles, et la seconde branche est fermée
* une position d'injection, dans laquelle les première et seconde branches sont ouvertes et communiquent entre elles, et la troisième branche est fermée
* et une position d'arrêt, dans laquelle les trois branches du distributeur sont fermées.

En pratique, un tel distributeur comporte un corps cylindrique dans lequel débouchent trois conduits disposés à 120° les uns par rapport aux autres, et correspondant aux trois branches décrites précédemment. Ce corps comporte un alésage cylindrique, dans lequel est disposé de manière rotative et coaxialement un noyau manoeuvrable par l'utilisateur grâce à un organe solidaire et extérieur de préhension, et comportant un conduit interne le traversant de part en part, avec deux branches disposées à 120° l'une de l'autre. C'est la rotation du noyau, dans quatre positions angulairement décalées, qui établit les trois modes de communication distincts, correspondant respectivement aux positions de purge, recueil, injection et arrêt.

Un tel distributeur comporte différents inconvénients.

On laisse à l'utilisateur ou au patient le soin de régler lui-même la position angulaire du noyau, par rapport au corps. S'agissant d'une personne inattentionnée ou handicapée, ceci rend la commande du distributeur dans l'une quelconque des positions fonctionnelles décrites précédemment, imprécise, aléatoire, voire erronée. L'utilisateur peut, en particulier, se tromper de sens de rotation, et il ne dispose d'aucun repère d'indexation pour identifier la position angulaire du noyau, correspondant au mode recherché de communication entre les branches du distributeur. Ceci est inacceptable pour un système de connexion destiné à soigner un malade, ou utilisé à des fins médicales ou thérapeutiques.

Un tel distributeur rotatif ne permet pas, d'une part d'isoler de manière étanche une branche par rapport aux autres, et d'autre part, d'isoler chaque branche, dans la position d'arrêt du distributeur. Ceci résulte du fait que la rotation du noyau suppose un jeu fonctionnel entre le corps et le noyau, par exemple avec deux joints toriques parallèles, par lequel une communication résiduelle entre les branches peut s'établir. Autrement dit, obtenir une étanchéité effective vis-à-vis de chaque branche est antinomique avec la rotation et la manoeuvre du noyau.

La présente invention a pour objet de remédier aux inconvénients précités. Plus précisément, l'invention a pour objet un système de connexion, dont la manoeuvre est particulièrement simple et fiable, en permettant une véritable étanchéité entre les différentes branches dudit système.

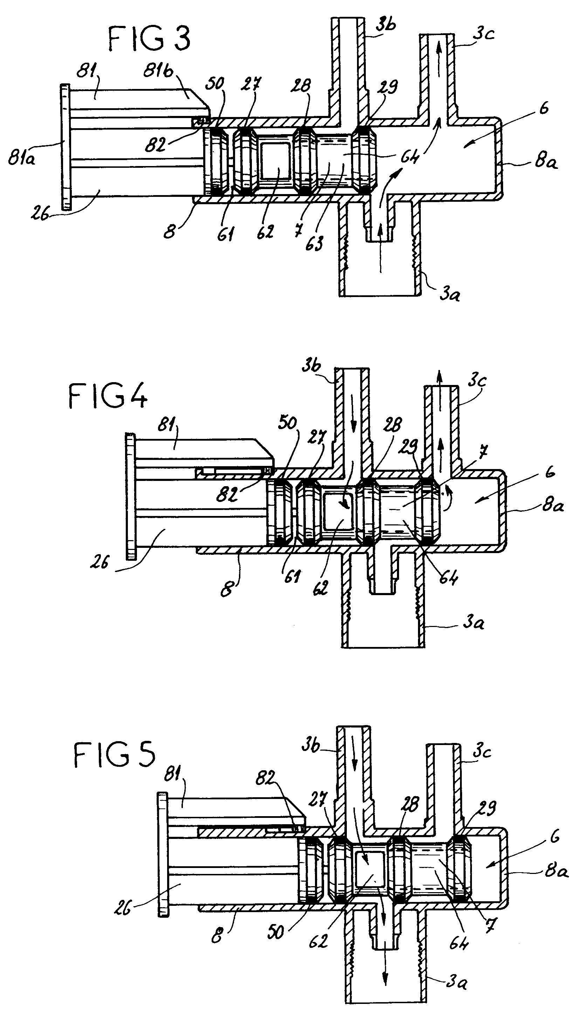

Selon l'invention, le distributeur retenu est un distributeur à tiroir, dont les différentes positions en translation par rapport au corps dudit distributeur, déterminent les positions de purge, recueil, et d'injection, ces trois positions sont obtenues par butée en translation du tiroir ou d'un élément solidaire dudit tiroir, et le distributeur à tiroir comprend :
- un corps comprenant au moins une paroi transversale de fermeture
- un tiroir, disposé à l'intérieur du corps cylindrique, associé à une tige de commande passant dans une ouverture du corps, comportant au moins trois nervures transversales, définissant entre elles au moins deux interstices, et
- un passage interne réalisé dans le tiroir entre le second interstice et la face postérieure ouverte du tiroir.
Grâce à l'invention, et comme montré par référence aux figures 3 à 7, il devient possible d'aligner selon l'axe du distributeur les différentes positions de recueil, purge, injection et arrêt, y compris de les ordonner selon cet axe, dans l'ordre des fonctions recherchées successivement pour le fonctionnement du système de connexion. Dans ce dernier cas, la manoeuvre ne consiste plus qu'à enfoncer, par crans successifs, le poussoir par rapport au corps.

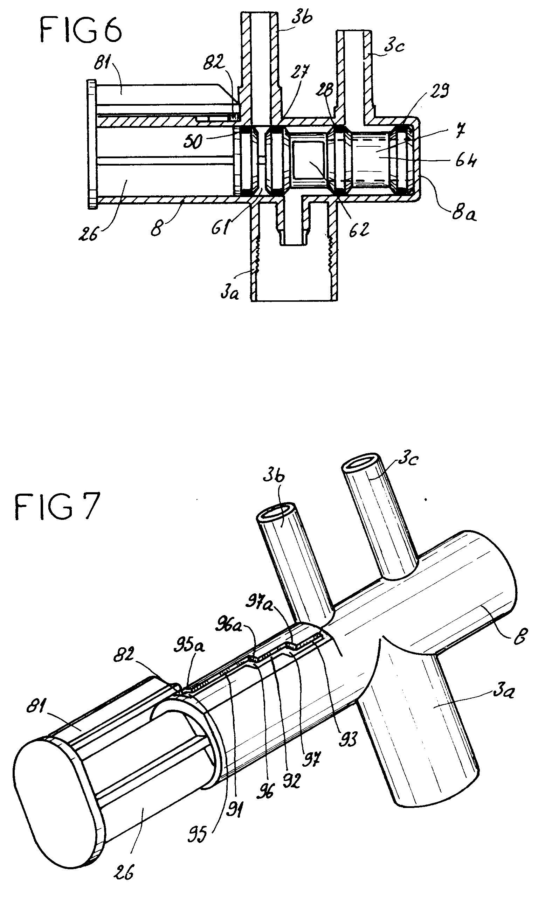

Grâce à l'invention, il devient particulièrement simple d'indexer les différentes positions prédéterminées du poussoir, en relation avec les différentes positions fonctionnelles identifiées précédemment. Comme montré par la figure 7, un simple doigt en relation avec une nervure appropriée suffit. Mais aussi le corps du distributeur, et ses parois de fond peuvent être utilisées aux mêmes fins.

Grâce à l'invention, le coulissement étanche du tiroir par rapport au corps, à la manière d'un piston, permet une vraie étanchéité entre les branches, et d'isoler complètement chacune d'entre elles, si nécessaire.

La présente invention est maintenant décrite par référence aux dessins annexés, dans lesquels :
* la figure 1 représente le schéma de principe d'un système de connexion stérile selon l'invention, déjà décrit dans le préambule de la présente description
* la figure 2 représente de manière schématique le système de connexion stérile selon l'invention
* les figures 3 à 6 représentent un mode d'exécution de l'invention, respectivement dans ses positions de recueil, purge, injection et arrêt ; selon ces figures, les connecteurs et autres branchements ne sont pas représentés
* la figure 7 représente le système de connexion selon figures 3 à 6, en perspective.

De manière générale, le système de connexion stérile selon l'invention répond à la description déjà faite par référence à la figure 1, aux exceptions près suivantes :
- les robinets 18, 20 et 22 sont supprimés
- l'organe de liaison 3 se confond avec le distributeur 6 décrit ci-après
- un joint 25 ruptible par pression obture la sortie de la poche 4 de soluté.

Le distributeur 7 forme avec le deuxième connecteur 2 un seul et même ensemble monobloc. Ses trois voies se confondent avec les trois branches 3a, 3b et 3c décrites respectivement, et reliées respectivement aux tuyaux, 19 vers la poche 4 de soluté, 21 vers la poche 5 de recueil de dialysat, et vers le second connecteur 2.

Ce distributeur est constitué par :
- un corps creux cylindrique 8 comportant une paroi transversale de fermeture 8a, située à une extrémité du corps 8
- le tiroir 7, ayant lui aussi la forme d'un cylindre, en ce qui concerne sa partie demeurant à l'intérieur du corps 8; le corps cylindrique du tiroir comporte trois nervures annulaires 27 à 29, espacées l'une de l'autre, en contact étanche chacune avec le corps cylindrique 8
- une tige 26 ou queue de commande en translation, monobloc avec le tiroir 7, se terminant par une partie de préhension, ou bouton de commande.

Conformément aux figures 3 à 6, on décrit chacune des positions prédéterminées du tiroir 7, par rapport au corps 8 du distributeur, en partant de la position initiale de purge représentée par la figure 4.

Conformément à la figure 4, la première branche 3a est fermée par des moyens décrits ci-après, et les seconde 3b et troisième 3c branches sont ouvertes et communiquent entre elles. Plus précisément la branche 3b communique avec l'interstice situé entre les deux nervures annulaires 28 et 29 du tiroir 7, lequel communique à son tour avec la troisième branche 3c.

Dans la position de recueil, les première 3a et troisième 3c branches sont ouvertes et communiquent entre elles, tandis que la seconde branche 3b demeure fermée. Plus précisément, l'interstice fermé entre les nervures annulaires 28 et 29 du tiroir 7 débouche sur la seconde branche 3b, tandis que ce tiroir 7 est en retrait par rapport au passage direct entre la première 3a et la troisième voie 3c.

Conformément à la figure 5, on passe à la position d'injection, en repoussant vers la droite le tiroir 7. Dans cette position, les première 3a et seconde 3b branches sont ouvertes et communiquent entre elles, tandis que la troisième branche 3c demeure fermée.

Plus précisément, la deuxième branche 3b communique avec la première branche 3a au travers de l'interstice existant entre les deux nervures annulaires 27 et 28 du tiroir 7, tandis que la branche 3c débouche sur l'interstice fermé entre les nervures 28 et 29 du tiroir 7.

Conformément à la figure 6, à partir de la position d'injection précédemment décrite, on passe à la position d'arrêt complet du système. Dans cette position d'arrêt, toutes les branches 3a, 3b et 3c se trouvent fermées.

Le tiroir 7 comporte une patte 81 s'étendant parallèlement et à distance de celui-ci, d'une extrémité 81a aplatie, solidaire de l'extrémité libre dudit tiroir, à une extrémité active 81b, en vis-à-vis du corps 8 du distributeur. Cette extrémité active 81b comporte un doigt 82, pouvant être engagé ou s'engageant dans trois entailles 95, 96, 97, ménagées à partir de la surface extérieure du corps 8, correspondant respectivement aux positions de recueil, purge et injection, progressivement décalées selon l'axe 2, de l'extrémité antérieure vers l'extrémité postérieure du corps 8, ainsi que décalées angulairement autour de l'axe de ce dernier, dans le même sens de rotation, c'est-à-dire dans le sens horaire selon la figure 7. Les deux flancs en vis-à-vis, référencés par la lettre (a) selon la figure 7, des entailles 95 à 97 respectivement, transversales et d'extension circulaire, déterminent trois contre-butées pour le doigt 82, en correspondance avec les trois positions du tiroir 7, représentées respectivement aux figures 3 à 5. Les trois entailles 95 à 97 sont reliées entre elles par des entailles axiales 91, 92 et 93, déterminant ensemble une rainure continue de la première entaille transversale 95a à la dernière entaille axiale 93, dans laquelle est engagée en permanence le doigt 82. Ce dernier arrivant en butée dans l'une quelconque des entailles axiales 95 à 97, peut être déplacé dans cette dernière, par rotation du tiroir 7, ce qui verrouille celui-ci dans l'une quelconque des positions représentées aux figures 3 à 5, et correspondant respectivement au recueil, à la purge, et à l'injection. Ce sont les entailles axiales 91 à 93 qui permettent le passage d'une position à une autre du tiroir 7 par rapport au corps 8.

Comme montré par les figures 3 à 6, le tiroir 7 est agencé, en correspondance avec le corps 8 du distributeur, et ses trois branches 3a à 3c, pour déterminer quatre positions du tiroir, selon figures 3 à 6 respectivement, dans lesquelles celui-ci est respectivement et progressivement enfoncé dans le corps 8 de la vanne, ceci permettant une ergonomie de manipulation du système de connexion, particulièrement simple et sans risque d'erreur, en relation avec le moyen de commande constitué, comme décrit précédemment, par la coopération du doigt 82 dans la nervure extérieure 95, 91, 96, 92, 97 et 93.

Outre les nervures 27 à 29, appelées ci-après respectivement première nervure, deuxième nervure et troisième nervure, le tiroir 7 comporte une quatrième nervure 50. Chacune de ces nervures est en pratique réalisée sous la forme d'une gorge dans laquelle est introduit et rapporté un joint torique. Toutes ces nervures déterminent, de l'extrémité antérieure à l'extrémité postérieure du tiroir 7, un premier interstice 61, un second interstice 62, et un troisième interstice 63. Le tiroir 7 se caractérise quant à lui par la présence d'un passage interne 64 entre le second interstice 62 et la face postérieure ouverte du tiroir 7.

La position axiale des nervures 27 à 29 et 50 sur le tiroir 7, par rapport aux branches 3a à 3c, est choisie de manière à ce que :
- dans la position de recueil (figure 3), la deuxième branche 3b est isolée des autres par la troisième nervure 29, et vers l'extérieur par la deuxième nervure 28
- dans la position de purge (figure 4), la première branche 3a est isolée des autres par les deuxième et troisième nervures 28 et 29, tandis que les branches 3b et 3c sont isolées vers l'extérieur, respectivement par la première nervure 27, et la paroi de fond 8a du distributeur ; dans cette position, le passage interne 64 fait communiquer les deuxième 3b et troisième 3c branches
- dans la position d'injection (cf figure 5), la troisième branche 3c est isolée des autres par la deuxième nervure 28, tandis que les branches 3b et 3a sont isolées de l'extérieur par la première nervure 27
- dans la position d'arrêt (cf figure 6), la première branche 3a est isolée par les nervures 27 et 28, et la nervure 29 vis-à-vis du passage 64 ; la deuxième branche 3b est isolée par les nervures 27 et 50 ; et la troisième branche 3c est isolée par les nervures 28 et 29.

## Revendications

1. Système de connexion stérile comprenant :
- un premier connecteur (1) destiné à être implanté et relié à la cavité péritonéale d'un patient
- un deuxième connecteur (2) agencé de manière complémentaire au premier connecteur, pour établir de manière réversible une liaison avec ce dernier
- un organe de liaison (3) consistant en un distributeur (6), à trois branches (3a,3b,3c) reliées respectivement au deuxième connecteur (2), à une réserve (4) et à un réservoir (5), ledit distributeur étant susceptible de prendre au moins trois positions, à savoir :
* une position de purge (figure.3), dans laquelle les seconde (3b) et troisième (3c) branches sont ouvertes et communiquent entre elles, et la première branche (3a) est fermée
* une position de recueil (figure.4), dans laquelle les première (3a) et troisième (3c) branches sont ouvertes et communiquent entre elles, et la seconde branche (3c) est fermée
* une position d'injection (figure.5), dans laquelle les première (3a) et seconde (3b) branches sont ouvertes et communiquent entre elles, et la troisième branche (3c) est fermée
**caractérisé en ce que** le distributeur (6) est un distributeur à tiroir, dont les différentes positions en translation de ce dernier déterminent les positions de purge, de recueil, et d'injection du distributeur, en ce que ces trois positions sont obtenues par butée en translation du tiroir (7) ou d'un élément solidaire dudit tiroir, et en ce que le distributeur à tiroir comprend :
- un corps (8) comprenant au moins une paroi transversale de fermeture (8a),
- un tiroir (7), disposé à l'intérieur du corps cylindrique, associé à une tige de commande (26) passant dans une ouverture du corps (8), comportant au moins trois nervures transversales (27 à 29,50), définissant entre elles au moins deux interstices, et
- un passage interne (64) réalisé dans le tiroir (7) entre le second interstice (62) depuis la face postérieure et la face postérieure ouverte du tiroir (7).

2. Système selon la revendication 1, caractérisé en ce que le tiroir (7) comporte une patte (81) s'étendant parallèlement et à distance dudit tiroir, d'une extrémité (81a) solidaire de l'extrémité libre de ce dernier, à une extrémité active (81b) en vis-à-vis du corps (8) du distributeur, et au moins les trois positions de recueil, purge et injection sont obtenues par butée de l'extrémité libre (81b) de la patte (81) contre trois contre-butées (95a à 97a) ménagées sur le corps (8), et correspondant aux trois positions du tiroir (7) respectivement.

3. Système selon la revendication 2, caractérisé en ce que l'extrémité active (81b) de la patte (81) comporte un doigt (82) s'engageant dans au moins une entaille transversale d'extension circulaire (95,96,97), ménagée à partir de la surface extérieure du corps (8), et dont au moins un flanc (95a,96a,97a) détermine une contre-butée pour le doigt (82).

4. Système selon la revendication 1, caractérisé en ce que le tiroir (7) est agencé en correspondance avec le corps (8) du distributeur, et ses trois branches (3a) à (3c), pour déterminer au moins trois positions du tiroir, dans lesquelles ce dernier est respectivement et progressivement enfoncé dans le corps (8) de la vanne, et correspondant respectivement aux positions de recueil, purge et injection.

5. Système selon les revendications 2 à 4, caractérisé en ce que le corps (8) comporte au moins trois entailles (95 à 97), correspondant respectivement aux positions de recueil, purge et injection, progressivement décalées selon l'axe du distributeur, de l'extrémité antérieure vers l'extrémité postérieure du corps (8) du distributeur.

6. Système selon la revendication 5, caractérisé en ce que les trois entailles (95 à 97) sont décalées angulairement autour de l'axe du corps (8), dans le même sens de rotation.

7. Système selon la revendication 6, caractérisé en ce que les trois entailles (95 à 97) sont reliées entre elles par des entailles axiales (91 à 93), déterminant ensemble une rainure continue de la première entaille axiale (95a) à la dernière entaille axiale (93), dans laquelle est engagé en permanence le doigt (82).

8. Système selon la revendication 1, caractérisé en ce que la position axiale des nervures (27,28,29,50) sur le tiroir (7), par rapport aux branches (3a,3b,3c) est telle que :
- dans la position de recueil, la deuxième branche (3b) est isolée des autres par la troisième nervure (29)
- dans la position de purge, la première branche (3a) est isolée des autres par les deuxième et troisième nervures (28,29), et le passage interne (64) fait communiquer les deuxième et troisième branches
- dans la position d'injection, la troisième branche (3c) est isolée des autres par les deuxième et troisième nervures (28).

9. Système selon l'une quelconque des revendications 1 à 8, caractérisé en ce qu'il comprend, d'un côté le premier connecteur (1), et de l'autre côté, de manière échangeable, notamment jetable, l'ensemble constitué par le deuxième connecteur (2), l'organe (3) de liaison avec son distributeur (6) à tiroir, relié au deuxième connecteur (2), la réserve (4) et le réservoir (5) respectivement reliés à l'organe de liaison.

10. Système selon la revendication 1, de dialyse ambulatoire, caractérisé en ce que le premier connecteur (1) est destiné à être relié à un drain implanté dans la cavité péritonéale du patient, la réserve pleine (4) contient une solution de dialyse, et le réservoir vide (5) est destiné à recueillir le dialysat.

## Claims

1. Sterile connection system comprising :
- a first connector (1) intended to be implanted in and linked to the peritoneal cavity of a patient
- a second connector (2) arranged in a complementary manner to the first connector, for establishing a connection with the latter in a reversible manner
- a connecting device (3) consisting of a distributor (6), with three branches (3a,3b,3c) connected respectively to the second connector (2), to a reservoir (4) and to a receiver (5), the said distributor being able to take up at least three positions, namely :
* a purge position (figure 3), in which the second (3b) and third (3c) branches are open and communicate with each other, and the first branch (3a) is closed
* a receiving position (figure 4), in which the first (3a) and third (3c) branches are open and communicate with each other, and the second branch (3c) is closed
* an injection position (figure 5), in which the first (3a) and the second (3b) branches are open, and communicate with each other, and the first branch (3c) is closed
**characterised in that** the distributor (6) is a slide distributor, of which the various positions of the latter in translation determine the purge, receiving and injection positions of the distributor, in that these three positions are obtained by stopping the translation of the slide (7) or a Component attached to the said slide, and in that the slide distributor comprises :
- a body (8) comprising at least one transverse closing wall (8a)
- a slide (7), disposed within the cylindrical body, associated with a control rod (26) passing through an opening in the body (8), comprising at least three transverse ribs (27 to 29,50), defining between them at least two interstices, and
- an internal passage (64) made in the slide (7) between the second interstice (62) from the rear face and the open rear face of the slide (7).

2. System according to claim 1, characterized in that the slide (7) comprises a lug (81) extending parallel to and at a distance from the said slide, from one end (81a) attached to the free end of the latter, to an active end (81b) facing the body (8) of the distributor, and at least three receiving, purge and injection positions are obtained by stopping the free end (81b) of the lug (81) against three counter stops (95a to 97a) arranged on the body (8), and corresponding to the three positions of the slide (7) respectively.

3. System according to claim 2, characterized in that the active end (81b) of the lug (81) comprises a finger (82) engaging in at least one transverse recess (95,96,97) extending circularly, made in the external surface of the body (8), and of which at least one flank (95a,96a,97a) forms a counter-stop for the finger (82).

4. System according to claim 1, characterized in that the slide (7) is arranged to correspond with the body (8) of the distributor, and its three branches (3a) to (3c), to determine at least three positions of the slide, in which the latter is respectively and progressively pressed into the body (8) of the valve, and corresponding respectively to the receiving purge, and injection positions.

5. System according to claims 2 to 4, characterized in that the body (8) comprises at least three recesses (95 to 97), corresponding respectively to the receiving, purge and injection positions, progressively offset along the axis of the distributor, from the front end to the rear end of the body (8) of the distributor.

6. System according to claim 5, characterized in that the three recesses (95 to 97) are offset angularly around the axis of the body (8), in the same direction of rotation.

7. System according to claim 6, characterized in that the three recesses (95 to 97) are connected to each other by axial recesses (91 to 93) forming together a continuous groove from the first axial recess (95a) to the final axial recess (93), in which the finger (82) is permanently engaged.

8. System according to claim 1, characterized in that the axial position of the ribs (27,28,29,50) on the slide (7), with respect to the branches (3a,3b,3c) is such that :
- in the receiving position, the second branch (3b) is isolated from the others by the third rib (29)
- in the purge position, the first branch (3a) is isolated from the others by the second and third ribs (28,29), and the internal passage (64) brings the second and third branches into communication.
- in the injection position, the third branch (3c) is isolated from the others by the second and third ribs (28).

9. System according to any one of claims 1 to 8, characterized in that it comprises, on the one hand the first connector (1) and on the other hand, in an exchangeable manner, in particular a disposable manner, the assembly consisting of the second connector (2), the connecting device (3) with its slide distributor (6), connected to the second connector (2), the reservoir (4) and the receiver (5), linked respectively to the connecting device.

10. System according to claim 1, for ambulatory dialysis, characterised in that the first connector (1) is intended to be connected to a drain implanted in the peritoneal cavity of the patient, the full reservoir (4) contains a dialysis solution, and the empty receiver (5) is intended to receive the dialysate.

## Patentansprüche

1. Steriles Verbindungssystem, welches aufweist:
- ein erstes Verbindungsstück (1) zur Implantierung, das mit der Bauchfellhöhle eines Patienten verbunden ist,
- ein zweites Verbindungsstück (2), das komplementär zu dem ersten Verbindungsstück ausgelegt ist, um mit letzterem eine reversible Verbindung herzustellen,
- ein aus einem Verteiler (6) bestehendes Bindeglied (3) mit drei Zweigen (3a, 3b, 3c), die mit dem zweiten Verbindungsstück (2), einem Vorrat (4) sowie einem Speicherbehälter (5) jeweils verbunden sind, wobei der Verteiler mindestens drei Stellungen einnehmen kann, nämlich:
* eine Spülstellung (Fig. 3), bei welcher der zweite Zweig (3b) und der dritte Zweig (3c) offen sind und miteinander in Verbindung stehen und der erste Zweig (3a) geschlossen ist,
* eine Auffangstellung (Fig. 4), bei welcher der erste Zweig (3a) und der dritte Zweig (3c) offen sind und miteinander in Verbindung stehen und der zweite Zweig (3c) geschlossen ist,
* eine Einleitstellung (Fig. 5), bei welcher der erste Zweig (3a) und der zweite Zweig (3b) offen sind und miteinander in Verbindung stehen und der dritte Zweig (3c) geschlossen ist,
**dadurch gekennzeichnet, daß** der Verteiler (6) ein Schiebeverteiler ist, dessen unterschiedliche Verschiebungsstellungen die Spül-, Auffang- und Einleitstellung des Verteilers bestimmen, daß die drei Stellungen mittels eines Verschiebungsanschlags des Schiebers (7) oder eines mit dem Schieber einstückigen Elements erhalten werden und daß der Schiebeverteiler aufweist:
- ein Gehäuse (8), das mindestens eine Verschlußwand in Querrichtung (8a) aufweist,
- einen Schieber (7), der im Innern des zylindrischen Gehäuses angeordnet ist und einer Steuerstange (26) zugeordnet ist, die in einer Öffnung des Gehäuses (8) verläuft, wobei der Schieber mindestens drei Rippen in Querrichtung (27 bis 29, 50) aufweist, die zwischen ihnen mindestens zwei Zwischenräume bilden, und
- einen inneren Durchtritt (64), der sich in dem Schieber (7), von der hinteren Seite aus betrachtet, zwischen dem zweiten Zwischenraum (62) und der hinteren offenen Seite des Schiebers (7) befindet.

2. System nach Anspruch 1, dadurch gekennzeichnet, daß der Schieber (7) eine Klaue (81) aufweist, die sich parallel zu und beabstandet von dem Schieber von einem mit dem freien Ende des Schiebers einstückigen Ende (81a) zu einem gegenüber dem Gehäuse (8) des Verteilers angeordneten aktiven Ende (81b) erstreckt, und daß mindestens die Auffang-, Spül- und Einleitstellung mittels eines Anschlags des freien Endes (81b) der Klaue (81) gegen drei auf dem Gehäuse (8) eingearbeitete Gegenanschläge (95a bis 97a) erhalten werden, die jeweils den drei Stellungen des Schiebers (7) entsprechen.

3. System nach Anspruch 2, dadurch gekennzeichnet, daß das aktive Ende (81b) der Klaue (81) eine Nase (82) aufweist, die mit mindestens einer kreisförmig verlaufenden Vertiefung in Querrichtung (95, 96, 97) in Eingriff ist, welche in die äußere Oberfläche des Gehäuses (8) eingearbeitet ist und bei der mindestens eine Flanke (95a, 96a, 97a) einen Gegenanschlag für die Nase (82) festlegt.

4. System nach Anspruch 1, dadurch gekennzeichnet, daß der Schieber (7) in Übereinstimmung mit dem Gehäuse (8) des Verteilers sowie seinen drei Zweigen (3a bis 3c) ausgelegt ist, um mindestens drei Stellungen des Schiebers festzulegen, in welchen er jeweils fortschreitend in das Gehäuse (8) des Schiebers eindringt und jeweils der Auffang-, Spül- und Einleitstellung entspricht.

5. System nach Anspruch 2 bis 4, dadurch gekennzeichnet, daß das Gehäuse (8) mindestens drei Vertiefungen (95 bis 97) aufweist, die jeweils der Auffang-, der Spül- und der Einleitstellung entsprechen und von dem Vorderende zu dem Hinterende des Gehäuses (8) des Verteilers fortschreitend entlang der Achse des Verteilers versetzt sind.

6. System nach Anspruch 5, dadurch gekennzeichnet, daß die drei Vertiefungen (95 bis 97) um die Achse des Gehäuses (8) in derselben Drehrichtung winkelmäßig versetzt sind.

7. System nach Anspruch 6, dadurch gekennzeichnet, daß die drei Vertiefungen (95 bis 97) untereinander durch axiale Vertiefungen (91 bis 93) verbunden sind, die miteinander eine durchgehende Rille von der ersten axialen Vertiefung (95a) bis zu der letzten axialen Vertiefung (93) festlegen, mit welcher die Nase (82) ständig in Eingriff ist.

8. System nach Anspruch 1, dadurch gekennzeichnet, daß die axiale Stellung der Rillen (27, 28, 29, 50) auf dem Schieber (7) bezüglich der Zweige (3a, 3b, 3c) derart ist, daß
- in der Auffangstellung der zweite Zweig (3b) von den anderen durch die dritte Rippe (29) isoliert ist,
- in der Spülstellung der erste Zweig (3a) von den anderen durch die zweite und dritte Rippe (28, 29) isoliert ist und der innere Durchtritt (64) den zweiten und den dritten Zweig miteinander verbindet,
- in der Einleitstellung der dritte Zweig (3c) von den anderen durch die zweite und die dritte Rippe (28, 29) isoliert ist.

9. System nach einem der Ansprüch 1 bis 8, dadurch gekennzeichnet, daß es auf der einen Seite das erste Verbindungsstück (1) und auf der anderen Seite austauschbar, insbesondere wegwerfbar, die Gruppe aufweist, welche aus dem zweiten Verbindungsstück (2), dem mit dem zweiten Verbindungsstück (2) verbundenen Bindeglied (3) mit seinem Schiebeverteiler (6), dem Vorrat (4) und dem Speicherbehälter (5) besteht, die jeweils mit dem Bindeglied verbunden sind.

10. System nach Anspruch 1, für die ambulante Dialyse, dadurch gekennzeichnet, daß das erste Verbindungsstück (1) zur Verbindung mit einer in die Bauchfellhöhle des Patienten implantierten Entwässerungsleitung dient, daß der volle Vorrat (4) eine Dialyselösung enthält und daß der leere Speicherbehälter (5) zum Auffangen des Dialysats dient.
